(19) Europäisches Patentamt
European Patent Office
Office européen des brevets

(11) EP 2 394 988 A1

(12) **EUROPEAN PATENT APPLICATION**
published in accordance with Art. 153(4) EPC

(43) Date of publication:
**14.12.2011 Bulletin 2011/50**

(21) Application number: **10738633.6**

(22) Date of filing: **08.02.2010**

(51) Int Cl.:
***C07C 279/22*** (2006.01) ***A61K 31/166*** (2006.01)
***A61K 31/192*** (2006.01) ***A61K 31/216*** (2006.01)
***A61K 31/277*** (2006.01) ***A61K 31/351*** (2006.01)
***A61K 31/426*** (2006.01) ***A61K 31/44*** (2006.01)
***A61P 25/18*** (2006.01) ***A61P 25/28*** (2006.01)
***A61P 43/00*** (2006.01) ***C07C 317/44*** (2006.01)
***C07C 323/62*** (2006.01) ***C07D 213/61*** (2006.01)
***C07D 277/20*** (2006.01) ***C07D 277/56*** (2006.01)
***C07D 309/22*** (2006.01)

(86) International application number:
**PCT/JP2010/051757**

(87) International publication number:
**WO 2010/090305 (12.08.2010 Gazette 2010/32)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **09.02.2009 JP 2009026855**

(71) Applicant: **Astellas Pharma Inc.**
**Tokyo 103-8411 (JP)**

(72) Inventors:
- **KINOYAMA, Isao**
**Tokyo 103-8411 (JP)**
- **KOGANEMARU, Yohei**
**Tokyo 103-8411 (JP)**
- **MIYAZAKI, Takehiro**
**Tokyo 103-8411 (JP)**
- **WASHIO, Takuya**
**Tokyo**
**1038411 (JP)**

(74) Representative: **HOFFMANN EITLE**
**Patent- und Rechtsanwälte**
**Arabellastraße 4**
**81925 München (DE)**

# (54) SUBSTITUTED ACYLGUANIDINE DERIVATIVE

(57) An object of the present invention is to provide an excellent agent for treating or preventing dementia, schizophrenia based on serotonin 5-$HT_{5A}$ receptor modulating action.

It was discovered that acylguanidine derivatives, in which the guanidine is bonded to one ring of a naphthalene via a carbonyl group and a cyclic group is bonded to the other ring thereof, exhibit potent the 5-$HT_{5A}$ receptor modulating action and excellent pharmacological actions based on the action. The present invention is useful as an excellent agent for treating or preventing dementia, schizophrenia, bipolar disorder or attention deficit hyperactivity disorder.

EP 2 394 988 A1

## Description

### Technical Field

The present invention relates to pharmaceuticals, particularly to substituted acyl guanidine derivatives with 5-$HT_{5A}$ receptor modulating action, useful as an agent for treating or preventing dementia, schizophrenia, and the like.

### Background Art

In recent years, it has been suggested that the 5-$HT_{5A}$ receptor which is one of the subtypes of serotonin receptors plays an important role in dementia and schizophrenia. For example, it has been reported that new exploratory behaviors are increased in the 5-$HT_{5A}$ receptor knock-out mice, and hyperactivity by LSD is inhibited in the 5-$HT_{5A}$ receptor knock-out mice (Neuron, 22, 581-591, 1999). From the results of gene expression analysis, it has been reported that the 5-$HT_{5A}$ receptor is highly expressed in human and rodent brain, and in brain, it is highly expressed in hippocampal CA1 and CA3 pyramidal cells which are related to memory, and frontal lobe (cerebral cortex) which is deeply related to schizophrenia (Molecular Brain Research, 56, 1-8,1998). Furthermore, it has been reported that gene polymorphism of the 5-$HT_{5A}$ receptor relates to schizophrenia (Neuroreport 11, 2017-2020, 2000; Mol. Psychiatr. 6, 217-219, 2001; and J. Psychiatr. Res. 38, 371-376, 2004). Accordingly, it is suggested that regulation of 5-$HT_{5A}$ receptor action leads to the improvement of dementia and schizophrenia and compounds with such function are needed.

Hitherto, several kinds of compounds having high affinity for the 5-$HT_{5A}$ receptor have been reported. For example, it has been described that a guanidine derivative represented by the following general formula binds to the 5-$HT_{5A}$ receptor and thus is used for treating multiple central diseases such as a neurodegenerative diseases and a neurophychiatric diseases (Patent Document 1).

[Chem. 1]

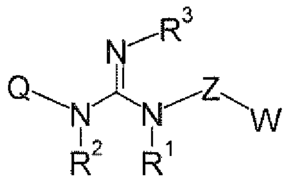

W: 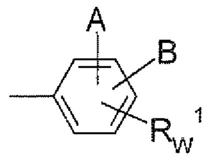 or 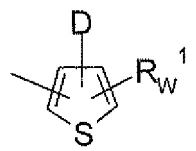

(A represents $NO_2$ $NH_2$ or the like, B represents a hydrogen atom or the like, $R_w^1$ represents a hydrogen atom or the like, D is a group represented by A, Q represents 2-substituted 5-membered heteroaryl, $R^1$, $R^2$ and $R^3$ represent a hydrogen atom or the like, and Z represents $-(CR_z^1R_z^2)_a-(V_z)_b-(CR_z^3R_z^4)_c-$, in which a and c are an integer of 0 to 4, b is an integer of 0 or 1, $R_z^1$, $R_z^2$, $R_z^3$ and $R_z^4$ represent a hydrogen atom or the like and $V_z$ represents CO or the like. For the details, refer to the the publication.)

None of the 5-$HT_{5A}$ receptor modulators which have been reported has a structure in which the guanidine is bonded to a naphthalene via a carbonyl group. On the other hand, several compounds having the aforesaid structure, which are used for other uses, are known.

For example, it has been reported that a derivative represented by the following general formula has an antiviral activity, and is useful in the treatment of HIV, HCV infections, and the like (Patent Document 2).

[Chem. 2]

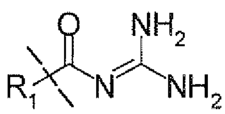

R1: 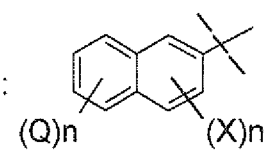 ,

and the like

($R^1$ represents phenyl, substituted phenyl, naphthyl, substituted naphthyl, or a structure shown above; n represents 1, 2, 3 or 4; Q independently represents hydrogen, cycloalkyl, thienyl, furyl, pyrazolyl, pyridyl, substituted pyridyl, phenyl,

substituted phenyl, or the like; and X represents hydrogen or alkoxy. For the details, refer to the publication.)
Furthermore, a patent application regarding a compound with similar structure has been filed by the present applicants (Patent Document 3). These publications have no description concerning the 5-$HT_{5A}$ receptor modulating action of the derivatives above, or their use for treating or preventing dementia, and schizophrenia.

In addition, naphthalene derivatives which exhibit inhibitory action on Na+/H+ exchange mechanisms and are useful for the treatment of myocardial infarction, angina pectoris or the like have been reported (Patent Documents 4 to 7 and Non-patent Document 1). None of these documents describes the 5-$HT_{5A}$ receptor modulating action of the naphthalene derivatives, or their use for treating dementia, or schizophrenia.

List of the Documents

Patent Document

Patent Document 1: WO 05/082871 pamphlet
Patent Document 2: WO 06/135978 pamphlet
Patent Document 3: WO 04/112687 pamphlet
Patent Document 4: U.S. Patent No. 6087304 Specification
Patent Document 5: U.S. Patent No. 6093729 Specification
Patent Document 6: Japanese Patent Publication JP-A-8-225513
Patent Document 7: U.S. Patent No. 5824691 Specification

Non-patent Document

Non-patent Document 1: Takeshi Yamamoto, et al., Chemical and Pharmaceutical Bulletin, Vol. 45, No. 8, p. 1282-1286, 1997.

Disclosure of the Invention

Problem that the Invention is to Solve

An object of the present invention is to provide an excellent agent for treating or preventing dementia, schizophrenia, or the like, based on the 5-$HT_{5A}$ receptor modulating action.

Means for Solving the Problem

As a result of intense research on compounds exhibiting 5-$HT_{5A}$ receptor modulating action, the present inventors discovered that acylguanidine derivatives in which the guanidine is bonded to the 2-position of a naphthalene via a carbonyl group and a cyclic group is bonded to the 8-position thereof, exhibit potent 5-$HT_{5A}$ receptor modulating action and therefore excellent pharmacological activities, and that they can be an agent for treating or preventing dementia, schizophrenia or the like, thereby completed the present invention.
That is, the present invention relates to compound of formula (I) or a pharmaceutically acceptable salt thereof.

[Chem. 3]

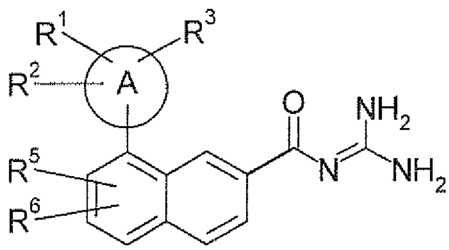

(I)

(wherein symbols have the following meanings:

[Chem. 4]

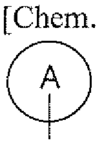

re

presents phenyl, cycloalkyl, monocyclic heteroaryl, or a saturated or partially unsaturated monocyclic oxygen-containing heterocyclic group;

$R^1$, $R^2$ and $R^3$ are the same as or different from each other, and represent H, lower alkyl, halogen, -CN, -$OR^a$, or lower alkylene-$OR^a$;

$R^a$ represents H or lower alkyl;

$R^5$ represents -CN, -SMe, or -$SO_nR^b$;

n represents 1 or 2;

$R^b$ represents lower alkyl; and

$R^6$ represents H or halogen.)

In this connection, unless otherwise specifically noted, when a symbol in a chemical formula is used in another chemical formula in this specification, the same symbols have the same meaning.

In addition, the present invention relates to a pharmaceutical composition containing compound of the aforesaid formula (I) or a pharmaceutically acceptable salt thereof and a pharmaceutically acceptable excipient; for example, the aforesaid pharmaceutical composition which is a 5-$HT_{5A}$ receptor modulator; in another embodiment, the aforesaid pharmaceutical composition which is an agent for preventing or treating dementia, schizophrenia, bipolar disorder or attention deficit hyperactivity disorder; in yet another embodiment, the aforesaid pharmaceutical composition which is an agent for preventing or treating dementia or schizophrenia.

Also, an embodiment of the present invention is use of compound of the aforesaid formula (I) or a pharmaceutically acceptable salt thereof for the manufacture of a 5-$HT_{5A}$ receptor modulator, for example, an agent for preventing or treating dementia, schizophrenia, bipolar disorder or attention deficit hyperactivity disorder, in particular, an agent for preventing or treating dementia or schizophrenia; in another embodiment, a method for preventing or treating dementia, schizophrenia, bipolar disorder or attention deficit hyperactivity disorder, in particular, a method for preventing or treating dementia or schizophrenia comprising administering a therapeutically effective amount of compound of the aforesaid formula (I) or a pharmaceutically acceptable salt thereof to a mammal.

## Effects of the Invention

Compounds of the present invention have an advantage of potent 5-$HT_{5A}$ receptor modulating action, and excellent pharmacological actions based on it, Thus, pharmaceutical compositions of the present invention is useful for treatment or prevention of 5-$HT_{5A}$ receptor-related diseases, and particularly, for prevention or treatment of dementia, schizophrenia, bipolar disorder, or attention deficit hyperactivity disorder.

## Modes for Carrying Out the Invention

Hereinafter, the present invention is described in more detail.

In the present specification, the "5-$HT_{5A}$ receptor modulator" is a generic term referring to a compound that inhibits activation of the 5-$HT_{5A}$ receptor by antagonizing with an endogenous ligand (5-$HT_{5A}$ antagonist), and a compound that shows function by continuous activation of the 5-$HT_{5A}$ receptor (5-$HT_{5A}$ agonist).

The "lower alkyl" is a linear or branched alkyl having 1 to 6 carbon atoms (hereinafter simply referred to as $C_{1-6}$), and specifically, methyl, ethyl, n-propyl, isopropyl, n-butyl, isobutyl, sec-butyl, tert-butyl, n-pentyl, n-hexyl groups, and the like. In another embodiment, it is $C_{1-4}$ alkyl, and in another embodiment, it is methyl, ethyl, n-propyl, and isopropyl groups.
The "lower alkylene" is linear or branched $C_{1-6}$ alkylene, and specifically, methylene, ethylene, trimethylene, tetramethylene, pentamethylene, hexamethylene, propylene, methylmethylene, ethylethylene, 1,2-dimethylethylene, 1,1,2,2-tetramethylethylene groups, and the like. In a further embodiment, it is $C_{1}$-$_{4}$ alkylene, and in another embodiment, it is methylene, ethylene, trimethylene, and propylene groups.

The "halogen" means F, Cl, Br, and I.
The "cycloalkyl" is a $C_{3}$-$_{10}$ saturated hydrocarbon ring group, which may have a bridge. Specifically, it is cyclopropyl, cyclobutyl, cyclopentyl, cyclohexyl, cycloheptyl, cyclooctyl, and adamantyl groups; in another embodiment, it is $C_{3}$-$_{6}$ cycloalkyl; in yet another embodiment, it is a cyclopropyl group.

The "monocyclic heteroaryl" refers to a 5- or 6-membered unsaturated ring group which contains 1 to 4 hetero atoms selected from oxygen, sulfur and nitrogen. Sulfur or nitrogen atoms which form the monocycle, may be oxidized and thus form oxide or dioxide. Specific examples thereof include pyridyl, pyrrolyl, pyrazinyl, pyrimidinyl, pyridazinyl, imidazolyl, triazolyl, thienyl, furyl, pyranyl, thiazolyl, pyrazolyl, isothiazolyl, oxazolyl, oxadiazolyl, isooxazolyl, and tetrazolyl groups. In another embodiment, it is pyridyl, pyrimidinyl, thienyl, thiazolyl, pyrazolyl, and oxadiazolyl groups. In another embodiment, it is pyridyl and thiazolyl groups. In yet another embodiment, it is a pyridyl group.

The "saturated or partially unsaturated monocyclic oxygen-containing heterocyclic group" refers to a 3- to 7-membered saturated or partially unsaturated monocyclic group which contains one oxygen atom, and may additionally contain one hetero atom selected from nitrogen, oxygen, and sulfur, among a heterocyclic group, and examples thereof include oxylanyl, oxetanyl, tetrahydrofuryl, tetrahydropyranyl, dihydropyranyl, and 1,4-dioxanyl groups. In another embodiment, it is a dihydropyranyl group.

Some embodiments of compound of formula (I) are shown below.

(1) The compound wherein,

[Chem. 5]

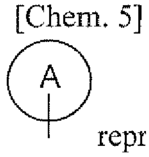

repr

represents phenyl, cyclopropyl, pyridyl, pyrimidinyl, thienyl, thiazolyl, pyrazolyl, oxadiazolyl, tetrahydropyranyl or dihydropyranyl group; in another embodiment, phenyl, cyclopropyl, pyridyl, thiazolyl, or dihydropyranyl group; in yet another embodiment, phenyl or pyridyl group.
(2) The compound wherein $R^1$, $R^2$, and $R^3$ are the same as or different from each other and represent H, methyl, F, Cl, CN, $-OR^a$ or $-CH_2OR^a$.
(3) The compound mentioned in (2), wherein $R^a$ represents H, methyl, or ethyl, and in another embodiment, H or methyl.
(4) The compound, wherein the substitution position of $R^5$ is the 5- or 7-position of the naphthalene.
(5) The compound wherein n represents 2.
(6) The compound wherein $R^b$ represents methyl.
(7) The compound wherein $R^6$ represents H, F, or Cl; in another embodiment, H or F.
(8) The compound with the groups mentioned in (1) and (2) above.
(9) The compound with the groups mentioned in (1), (2), and (4) above.
(10) The compound with the groups mentioned in (1), (2), (4), and (7) above.
(11) The compound with two or more of groups mentioned in any of the above (1) to (7).
(12) The compound selected from the group consisting of:

7-cyano-N-(diaminomethylene)-8-(2,4,6-trifluorophenyl)-2-naphthamide,
7-cyano-N-(diaminomethylene)-8-(2,4,6-difluorophenyl)-2-naphthamide,
5-cyano-N-(diaminomethylene)-7-fluoro-8-(2,4,6-trifluarophenyl)-2-naphthamide,
8-(2-chloro-6-ftuorophenyl)-7-cyano-N-(diaminomethylene)-2-naphthamide, and
7-cyano-N-(diaminomethylene)-8-(3,5-difluoropyridin-4-yl)-2-naphthamide,

or a salt thereof.

Compound of formula (I) may exist as other tautomers, geometrical isomers, or optical isomers, depending on the kind of the substituents. The present invention includes these isomers, isolated formes, or mixtures thereof.
Furthermore, pharmaceutically acceptable prodrugs of compound of formula (I) are also included in the present invention. Pharmaceutically acceptable prodrugs refer to compounds which have a group that can be converted into an amino group, OH, $CO_2H$, or the like by solvolysis or under physiological conditions, thus releasing compound of formula (I) *in vivo* after administration. Examples of the group forming prodrugs include the groups described in "Prog. Med., 5, 2157-2161 (1985), and "Iyakuhin no Kaihatsu (Development of Medicines)" (Hirokawa Publishing Company, 1990), vol. 7, Bunshi Sekkei (Molecular Deign)", 163-198.
Furthermore, compound of formula (I) may form an acid addition salt, or may form a salt with a base depending on the kind of substituents, and the salts are included in the present invention, as long as the they are pharmaceutically acceptable salts. Specifically, examples of these salts include acid addition salts with inorganic acids such as hydrochloric acid, hydrobromic acid, hydroiodic acid, sulfuric acid, nitric acid, and phosphoric acid, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, and glutamic acid, salts with inorganic bases such as sodium, potassium, magnesium, calcium, and aluminum, and organic bases such as methylamine, ethylamine, ethanolamine, lysine, and ornithine, and ammonium salts.
In addition, compound of formula (I) and a pharmaceutically acceptable salt thereof may exist as hydrates, solvates, and crystal polymorphs, and the present invention includes all of them. Also, compound of formula (I) and a pharmaceutically acceptable salt thereof include those labeled with radioactive or non-radioactive isotopes.

(Production Processes)

Compound of formula (I) and a pharmaceutically acceptable salt thereof can be produced by applying various known synthetic methods, utilizing its basic skeleton or type of substituents. Protection of functional groups with suitable protecting groups (a group which can be easily converted into the original functional group), may be effective in technical means, depending on the kinds of the functional groups, in any step from starting materials to intermediates. Examples of the functional groups include amino group, hydroxyl group, and carboxyl group, and examples of the protecting groups include those described in "Green's Protective Groups in Organic Synthesis (4th Edition, 2006)", edited by P. G. M. Wuts and T. W. Greene, which can be optionally selected and used depending on the reaction conditions. In this way, a desired compound can be obtained by introducing a protecting group to carry out the reaction, and then, removing the protecting group, if desired.
In addition, prodrugs of compound of formula (I) can be produced by introducing a specific group during any step from starting materials to intermediates, in a similar way to the aforementioned protecting groups, or by carrying out a reaction using the obtained compound of formula (I). The reaction may be carried out by employing a method known to a skilled person in the art, such as ordinary esterification, amidation, and dehydration.
Hereinbelow, representative production processes of compound of formula (I) are described. Each production process can be carried out according to the references cited in the description. Further, production processes of the present invention are not limited to the examples as shown below.

(General production process)

[Chem. 6]

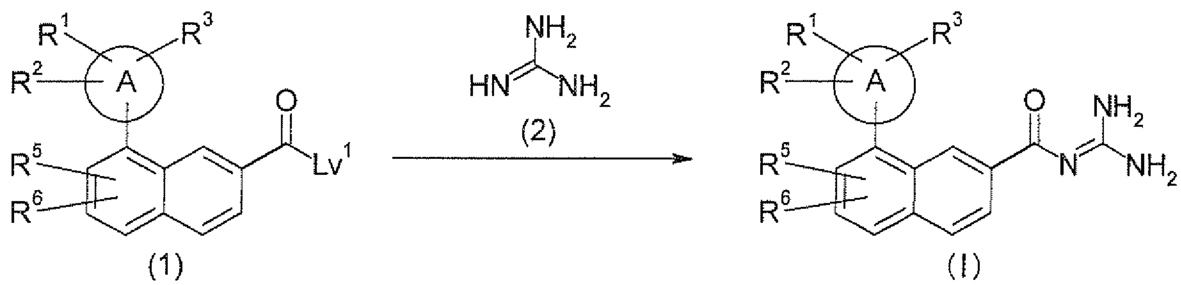

(1) (2) (I)

($Lv^1$ represents -OH or a leaving group.)

Compound of formula (I) can be produced by reaction of a carboxylic acid or a reactive derivative thereof (1) with guanidine (2) or a salt thereof.
The reaction can be carried out using equivalent amounts of the carboxylic acid or a reactive derivative thereof (1) and guanidine (2), or excess amount of guanidine. It can be carried out under cooling to under heating, preferably from -20°C to 80°C, in a solvent inert to the reaction; such as aromatic hydrocarbons such as benzene, toluene, or xylene; halogenated hydrocarbons such as dichloromethane, 1,2-dichloroethane, or chloroform; ethers such as diethylether, tetrahydrofuran (THF), dioxane, or dimethoxyethane (DME); N,N-dimethylformamide (DMF); dimethylsulfoxide (DMSO); N-methylpyrolidone (NMP); ethyl acetate; acetonitrile; or water; or mixtures thereof.
When carboxylic acid wherein $Lv^1$ is OH is used as starting compound (1), it is desirable to carry out the reaction in the presence of a condensing agent. In this case, examples of the condensing agents include N,N'-dicyclohexylcarbodiimide (DCC), 1-[3-(dimethylamino)propyl]-3-emylcarbodiimide (WSC), 1,1'-carbonyldiimidazole (CDI), 2-(1H-benzotriazol-1-yl)-1,1,3,3-tetramethyluronium hexafluorophosphate (HBTU), diphenylphosphoryl azide (DPPA), and phosphorous oxychloride. In some cases, it is preferable to further use additive agents (e.g., N-hydroxysuccinimide (HONSu), 1-hydroxybenzotriazole (HOBt), and the like). The condensing agent is usually used in an equivalent amount or excess to the carboxylic acid.
Examples of the reactive derivatives of the carboxylic acid wherein $Lv^1$ is a leaving group in starting compound (1), are acid halides (acid chloride, acid bromide, or the like), acid anhydrides (mixed acid anhydride with phenyl chlorocarbonate, p-toluenesulfonic acid, isovaleric acid, or the like or symmetric acid anhydrides), active esters (esters which can be prepared using phenol that may be substituted with an electron withdrawing group such as a nitro group or a fluorine atom, HOBt, HONSu and the like), lower alkyl esters. Each of them can be produced from carboxylic acid using reactions obvious to those skilled in the art. Addition of bases (organic bases such as triethylamine, diisopropylethylamine (DIPEA), N-rnethylrnorpholine, pyridine, or 4-(N,N-dimethylamino)pyridine, or inorganic bases such as sodium hydrogen carbonate, or the like) may be advantageous for smooth progress of the reaction, depending on the kinds of the reactive derivatives. Pyridine can also serve as a solvent. In this connection, when a lower alkyl ester is used as the reactive derivative, it is preferable to carry out the reaction from room temperature to refluxing with heating.
Starting compound (1) for general production process may be prepared by known methods or any variation thereof. For example, starting compound (1a) may be prepared in accordance with the following reaction scheme (production process of the starting compound).

(Production process of the starting compound)

[Chem. 7]

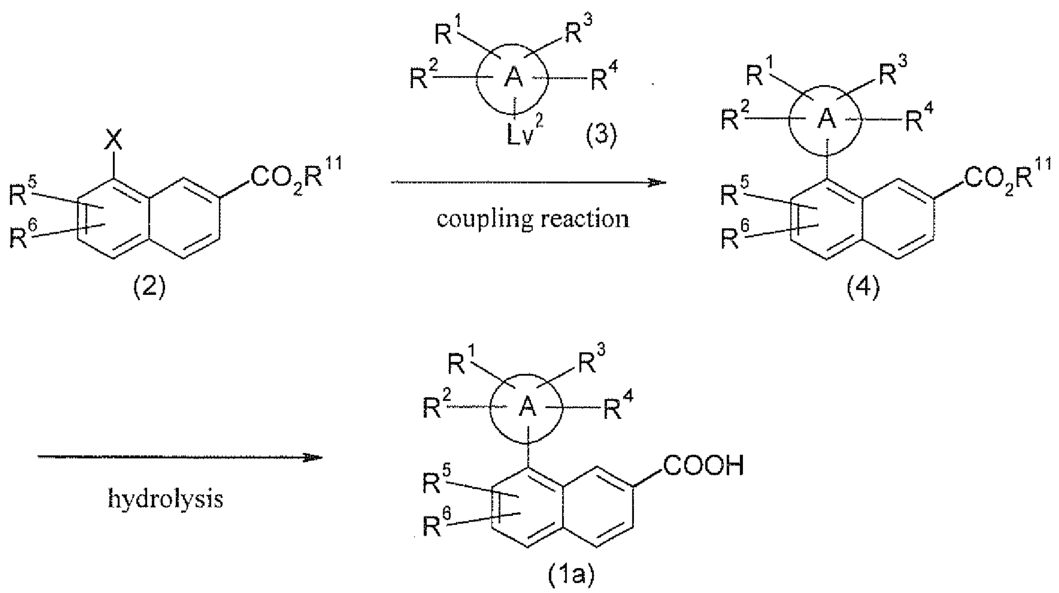

(In the formula, X represents trifluoromethanesulfonyloxy, $-B(OH)_2$ or -B(OZ)OW, $R^{11}$ represents a protecting group of a carboxyl group such as lower alkyl or benzyl, and $Lv^2$ represents a leaving group. Here, Z and W are the same as or different from each other and represent lower alkyl, or Z and W are combined together to form a lower alkylene.)
Compound (1a) may be obtained by coupling reaction of compound (2) with compound (3) to obtain compound (4) and hydrolyzing compound (4).
Examples of leaving groups represented by $Lv^2$ include halogen, methanesulfonyloxy, p-toluenesulfonyloxy, trifluoromefihanesulfonyloxy groups, and the like.
Compound (4) may be synthesized by stirring compound (2) and compound (3) in equivalent amounts or in excess amount of one of them; in a reaction inert solvent in the presence of a base and palladium catalyst at room temperature to refluxing with heating, usually for 0.1 hour to 5 days. This reaction is carried out preferably under an inert gas atmosphere. Examples of solvents used herein include, but are not particularly limited to, aromatic hydrocarbons, ethers, halogenated hydrocarbons, alcohols, DMF, DMSO, and mixed solvent thereof. As the bases, inorganic bases such as sodium carbonate, potassium carbonate and sodium hydroxide are preferred. As the palladium catalysts, tetrakis(triphenylphosphine)palladium, dichlorobis(triphenylphosphine)palladium, palladium-1,1'-bis(diphenylphosphino)ferrocene chloride and the like are preferred.
The coupling reaction may be carried out with reference to the following documents.

[Documents]

A. d. Meijere and F. Diederich et al., "Metal-Catalyzed Cross-Coupling Reactions", 1st edition, VCH Publishers Inc., 1997
The Chemical Society of Japan, "Jikken Kagaku Koza (Courses in Experimental Chemistry) 5th edition", Vol. 13 (2005) (Maruzen)

Subsequently, compound (4) is subjected to a hydrolysis reaction to obtain compound(Ia). The hydrolysis reaction may be carried out with reference to P. G M. Wuts and T. W. Greene, "Green's Protective Groups in Organic Synthesis (4th edition, 2006)".

(Other production processes)

In addition, the above described compounds (2) and (3) (Production process of the starting compound) may be prepared by known methods or any variation thereof, for example, in accordance with methods mentioned in the following Preparation Examples.
Compound of formula (I) prepared in accordance with the aforementioned methods is isolated and purified as a free compound, as a pharmaceutically acceptable salt thereof, as a hydrate or solvate thereof, or a crystalline polymorph thereof. Pharmaceutically acceptable salts of compound of formula (I) may be prepared using salt preparation methods well-known to those skilled in the art.
Isolation and purification are carried out by applying common chemical operations such as extraction, fractional crystallization and fractional chromatography.
A variety of isomers may be isolated by selecting suitable starting compounds or using differences in physicochemical properties among the isomers. For example, optical isomers may be led to stereochemically pure isomers by a general optical resolution method (for example, fractional crystallization to lead into diastereomer salts with an optically active base or acid, or chromatography using a chiral column). Also, it can be prepared from suitable optical active starting compounds.

Examples

Hereinafter, production processes of compound of formula (I) are described as Examples. Further, the production processes of compounds used as starting materials are described as Preparation Examples. The production processes of compound of formula (I) are not limited to production processes of the following specific Examples, but compounds of formula (I) may be prepared by combining these production processes or known production processes.

Preparation Example 1

A mixture of methyl 7-fluoro-8-oxo-5,6,7,8-tetrahydronaphthalene-2-carboxylate (9.21 g), copper (II) bromide (20.4 g), and THF (300 mL) was stirred at an oil temperature of 60°C for 6 days. The reaction mixture was cooled to

room temperature, the insoluble matter was separated by filtration, and the filtrate was concentrated under reduced pressure. The resulting residue was purified by silica gel column chromatography (hexane/ethyl acetate) to obtain methyl 7-bromo-7-fluoro-8-oxo-5,6,7,8-tetrahydronaphthalene-2-carboxylate (13.6 g).

Preparation Example 2

A mixture of methyl 7-bromo-7-fluaro-8-oxo-5,6,7,8-tetrahydronaphthalene-2-carboxylate (13.6 g), lithium bromide (7.8 g), and DMF (100 mL) was stirred with heating at an oil temperature of 100°C for 10 hours. The reaction mixture was cooled in ice, diluted with water, and the resulting precipitate was collected by filtration to obtain methyl 7-fluoro-8-hydroxy-2-naphthalene carboxylate (8.7 g).

Preparation Example 3

m-Chloroperbenzoic acid (content: about 70%, 1.05 g) was added at 0°C to a mixture of methyl 7-(methylsulfanyl)-8-(2,4,6-trifluorophenyl)-2-naphthalene carboxylate (220 mg) and dichloromethane (15 mL), followed by stirring for 16 hours. The reaction mixture was diluted with an aqueous sodium thiosulfate solution and extracted with ethyl acetate. The organic layer was concentrated under reduced pressure, and the resulting residue was purified by silica gel column chromatography (hexane/ethyl acetate) to obtain methyl 7-(methylsulfonyl)-8-(2,4,6-trifluorophenyl)-2-naphthalene carboxylate (221 mg).

Preparation Example 4

A mixture of methyl 7-cyano-8-(2,6-difluoro-4-formylphenyl)-2-naphthalene carboxylate (168 mg), sodium borohydride (18 mg), THF (10 mL), and methanol (20 mL) was stirred at room temperature for 3 hours. The reaction mixture was diluted with water and extracted with ethyl acetate. The organic layer was washed with water, dried, and concentrated under reduced pressure to obtain methyl 7-cyano-8-[2,6-difluo\ro-4-(hydroxy methyl)phenyl]-2-naphthalene carboxylate (227 mg).

Preparation Example 5

A mixture of methyl 7-cyano-8-(2,6-difluorophenyl)-2-naphthalene carboxylate (129 mg), a 1M aqueous sodium hydroxide solution (2 mL), THF (10 mL), and methanol (10 mL) was stirred at room temperature for 6 hours. The reaction mixture was concentrated under reduced pressure, the resulting residue was neutralized with 1M hydrochloric acid, and the precipitate was collected by filtration to obtain 7-cyano-8-(2,6-difluorophenyl)-2-naphthalenecarbonic acid (110 mg).

Preparation Example 6

Pyridinium bromide perbromide (702 mg) was added at 0°C to a mixture of methyl 7-fluoro-8-hydroxy-2-naphthalene carboxylate (460 mg), methanol (20 mL), and dichloromethane (20 mL), followed by stirring at room temperature for one hour. The reaction mixture was diluted with water and extracted with ethyl acetate. The organic layer was washed with water, dried, and concentrated under reduced pressure to obtain methyl 5-bromo-7-fluoro-8-hydroxy-2-naphthalene carboxylate (610 mg).

Preparation Example 7

A mixture of methyl 7-bromo-8-oxo-5,6,7,8-tetrahydronaphthalene-2-carboxylate (6.88 g), a methyl mercaptan sodium salt (1.7 g), and DMF (100 mL) was stirred at room temperature for 3 hours. The reaction mixture was diluted with water and extracted with ethyl acetate. The organic layer was concentrated under reduced pressure and the resulting residue was purified by silica gel column chromatography (hexane/ethyl acetate) to obtain methyl 7-(methylsulfanyl)-8-oxo-5,6,7,8-tetrahydronaphthalene-2-carboxylate (3.97 g).

Preparation Example 8

Trifluoromethanesulfonic anhydride (462 mg) was added to a mixture of methyl 7-cyano-8-hydroxy-2-naphthalene carboxylate (240 mg), triethylamine (171 mg), and dichloromethane (20 mL), followed by further stirring at room temperature for 17 hours. The reaction mixture was diluted with water and extracted with chloroform, and the organic layer was concentrated under reduced pressure. The resulting residue was purified by silica gel column chromatography (hexane/ethyl acetate) to obtain methyl 7-cyano-8-{[(trifluoromethyl)sulfonyl]oxy}-2-naphthalene carboxylate (280 mg).

Preparation Example 9

A mixture of methyl 7-bromo-8-hydroxy-2-naphthalene carboxylate (10.2 g), zinc cyanide (content of 60%, 7.1 g), tris(dibenzylideneacetone)dipalladium(0) (1.66 g), 1,1'-bis(diphenylphosphino)ferrocene (2.0 g), and N-methyl-2-pyrrolidone (100 mL) was stirred under heating at an oil temperature of 150°C for 3 hours. The reaction was cooled to room temperature, diluted with water and ethyl acetate, and insoluble matter was separated by filtration. The filtrate was subjected to liquid separation, the organic layer was concentrated under reduced pressure and the resulting residue was purified by silica gel column chromatography (hexane/ethyl acetate) to obtain methyl 7-cyano-8-hydroxy-2-naphthalene carboxylate (5.89 g).

Preparation Example 10

A mixture of methyl 7-cyano-8-{[(trifluoromethyl)sulfonyl]oxy}-2-naphthalene carboxylate (300 mg), bis(pinacolato)diboron (223 mg), bis(triphenylphosphine) palladium chloride (29 mg), triphenylphosphine (22 mg) and potassium acetate (246 mg), and 1,4-dioxane (5 mL) was stirred under heating at an oil temperature of 100°C for 18 hours. The reaction mixture was cooled to room temperature, the insoluble matter was separated by filtration, and the filtrate was concentrated under reduced pressure. The resulting residue was diluted with water and extracted with ethyl acetate and the organic layer was concentrated under reduced pressure. The resulting residue was purified by silica gel column chromatography (hexane/ethyl acetate) to obtain methyl 7-cyano-8-(4,4,5,5-tetramethyl-1,3,2-dioxaborolan-2-yl)-2-naphthalene carboxylate (181 mg).

Preparation Example 11

A mixture of methyl 8-oxo-5,6,7,8-tetrahydronaphthalene-2-carboxylate (3.0 g), 1-fluoro-4-hydroxy-1,4-diazoniabicyclo[2,2,2]octane bis(tetrafluoroborate) (5.2 g), and methanol (140 mL) was stirred under refluxing with heating for 3 hours. The reaction mixture was concentrated under reduced pressure, diluted with dichloromethane, and the insoluble matter was separated by filtration. The filtrate was concentrated under reduced pressure and the resulting residue was purified by silica gel column chromatography (hexane/ethyl acetate) to obtain methyl 7-fluoro-8-oxo-5,6,7,8-tetrahydronaphthalene-2-carboxylate (2.8 g).

Preparation Example 12

n-Butyl lithium (1.55M hexane solution, 7.5 mL) was added under an argon gas atmosphere at -78°C to a mixture of N,N,N',N'-tetramethylethylenediamine (1.5 g), and diethylether (40 mL), followed by stirring at the same temperature for 30 minutes. A mixture of 3,5-difluoropyridine (1.2 g) and diethylether (10 mL) was slowly added to the reaction mixture, followed by stirring at the same temperature for 2 hours. Iodine (4.0 g) was further added to the reaction mixture, followed by stirring at the same temperature for one hour and cooling to room temperature. The reaction mixture was diluted with water, the formed solid was separated by filtration, and the filtrate was extracted with diethyl ether and washed with a saturated aqueous sodium hydrogen carbonate solution. The organic layer was washed with saturated brine, dried over anhydrous magnesium sulfate, and concentrated under reduced pressure to obtain 3,5-difluoro-4-iodopyridine (820 mg).

Preparation Example 13

A mixture of methyl 7-cyano-8-{[(trifluoromethyl)sulfonyl]oxy}-2-naphthalene carboxylate (250 mg), 2,6-difluorophenyl boric acid (165 mg), [1,1'-bis(diphenylphosphino)ferrocene]dichloro palladium (II) (102 mg), cesium fluoride (211 mg), and 1,2-dimethoxyethane (15 mL) was stirred under refluxing with heating under an argon atmosphere for one day. The reaction mixture was cooled to room temperature, diluted with water and extracted with ethyl acetate. The organic layer was concentrated under reduced pressure and the resulting residue was purified by silica gel column chromatography (hexane/ethyl acetate) to obtain methyl 7-cyano-8-(2,6-difluorophenyl)-2-naphthalene carboxylate (134 mg).

Preparation Example 14

A mixture of methyl 7-cyano-8-{[(trifluoromethyl)sulfonyl]oxy}-2-naphthalene carboxylate (400 mg), 2,6-difluoro-4-formylphenylboronic acid pinacol ester (448 mg), palladium (II) acetate (25 mg), potassium triphosphate (994 mg), 2-dicyclohexylphosphino-2',4',6'-triisopropylbiphenyl (106 mg), and toluene (5 mL) was stirred under heating under an argon gas atmosphere at an oil temperature of 100°C for 3 hours. The reaction mixture was cooled to room temperature,

diluted with water and extracted with ethyl acetate. The organic layer was concentrated under reduced pressure and the resulting residue was purified by silica gel column chromatography (hexane/ethyl acetate) to obtain methyl 7-cyano-8-(2,6-difluoro-4-formylphenyl)-2-naphthalene carboxylate (168 mg).

The compounds of Preparation Examples shown in Tables 1 to 5 below were prepared using the corresponding starting materials in the same manner as in the above shown Preparation Examples 1 to 14. In addition, physical data for the compounds of Preparation Examples are shown in Tables 6 and 7.

Example 1

A mixture of 8-(2-chloro-6-fluorophenyl)-7-cyano-2-naphthalenecarbonic acid (75 mg), CDI (52 mg), and DMF (10 mL) was stirred under heating at an oil temperature of 60°C for 30 minutes, the reaction mixture was cooled to room temperature, and guanidine carbonate (104 mg) was added thereto, followed by further stirring for 15 hours. The reaction mixture was diluted with sodium bicarbonate aqueous solution , extracted with ethyl acetate, washed with an aqueous saturated sodium chloride solution and dried over anhydrous magnesium sulfate. The organic layer was concentrated under reduced pressure, and the resulting residue was purified by silica gel column chromatography (chloroform/methanol=20/1), further treated with a 4M hydrogen chloride/1,4-dioxane solution, and washed under heating with ethyl acetate, to obtain 8-(2-chloro-6-fluorophenyl)-7-cyano-N-(diaminomethylene)-2-naphthamide hydrochloride (40 mg).

The compounds of the Examples shown in the Tables 8 and 9 below were prepared using the corresponding starting materials in the same manner as in Example 1 above. The physical data for the compounds of the Examples are shown in Table 10.

The following abbreviations are used in the Tables below.

PEx: Preparation Example number, Ex: Example number, Str: structural formula, Dat: physical data (ESI+: ESI-MS $[M+H]^+$; ESI-: ESI-MS$[M-H]^-$; EI+: EI$[M]^+$; A/E+: simultaneous measurement of APCI and ESI (cations); A/E-: simultaneous measurement of APCI and ESI (anions); NMR: 6(ppm) of peaks by $^1$HNMR in $CDCl_3$ or DMSO-$d_6$); Sal: salt (empty column or no description represents a free form, and the numeral present before the acidic ingredient represents a molar ratio; for example, the case in which 2HCl is described shows that the compound is a dihydrochloride); Me: methyl; Tf: trifluoromethanesulfonyl; RSyn: production process (the numeral shows that, the compound was produced by the similar method as in the compound having the number as its Preparation Example number, using the corresponding starting material).

[Table 1]

| REx | Str |
|---|---|
| 1 | 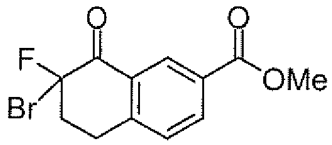 |
| 2 | 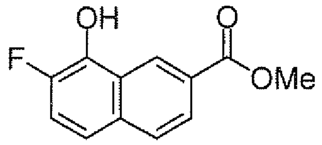 |
| 3 | 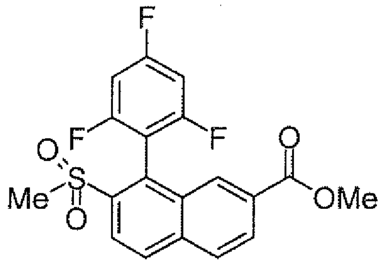 |
| 4 | 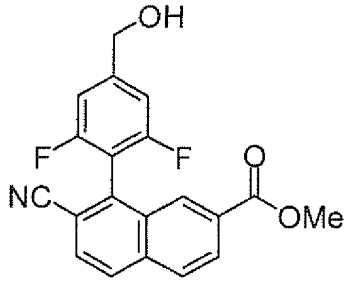 |
| 5 | 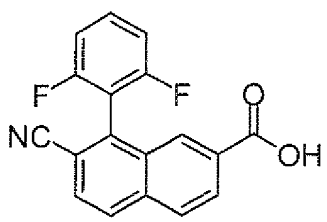 |
| 6 | 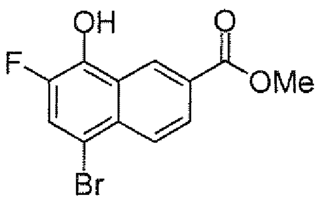 |
| 7 | 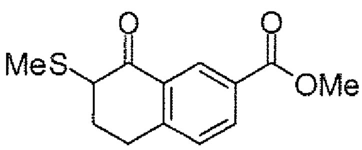 |
| 8 | 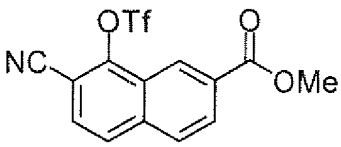 |
| 9 | 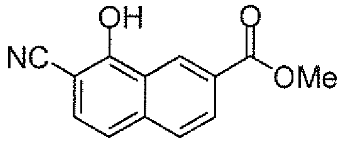 |
| 10 | 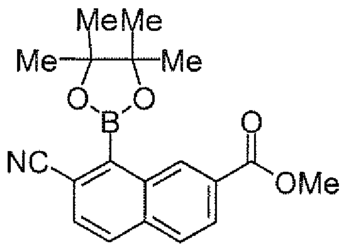 |
| 11 | 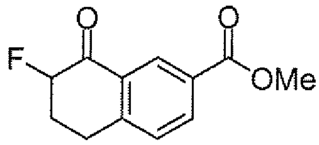 |
| 12 | 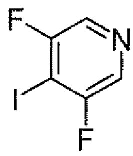 |
| 13 | 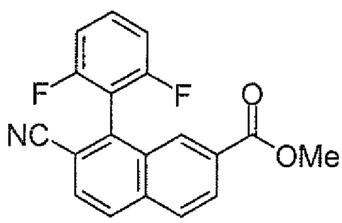 |
| 14 | 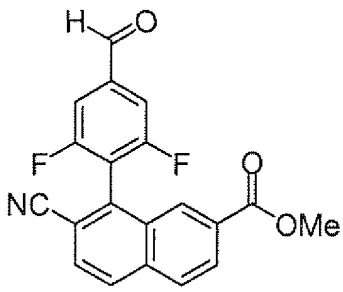 |

[Table 2]

| REx | Str |
|---|---|
| 15 | 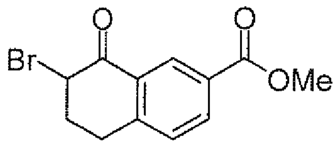 |
| 16 | 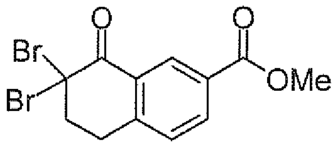 |
| 17 | 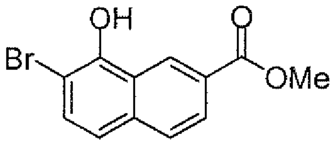 |
| 18 | 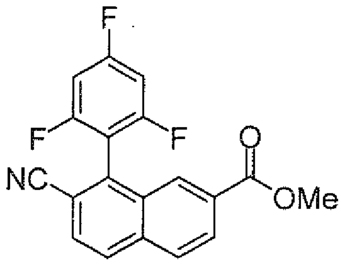 |
| 19 | 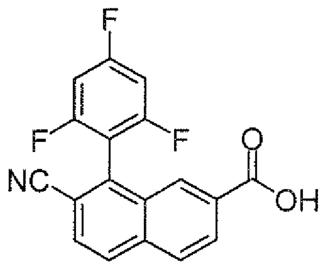 |
| 20 | 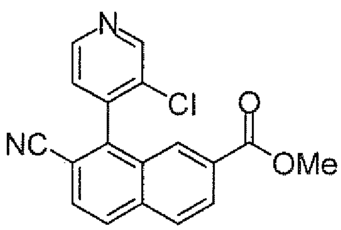 |
| 21 | 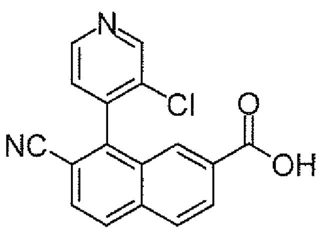 |
| 22 | 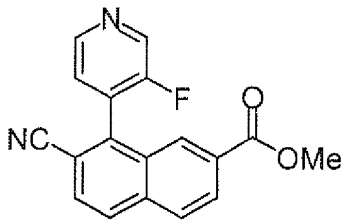 |
| 23 | 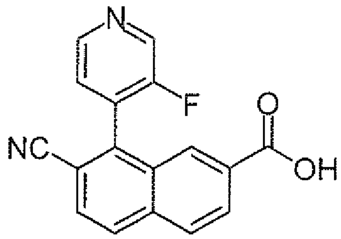 |
| 24 | 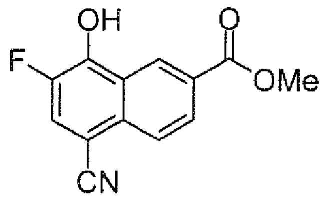 |
| 25 | 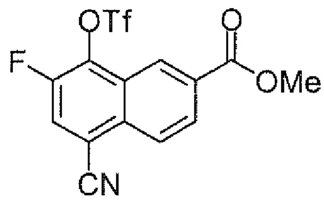 |
| 26 | 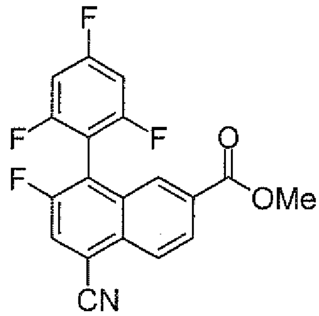 |
| 27 | 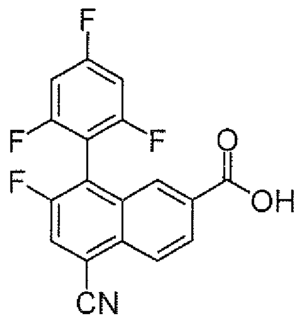 |

[Table 3]

| REx | Str |
|---|---|
| 28 | 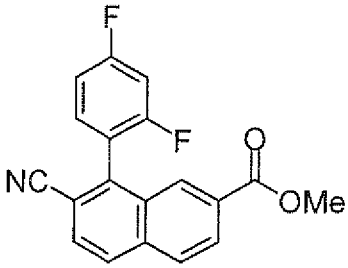 |
| 29 | 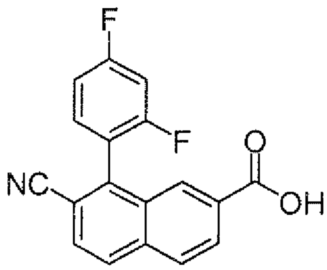 |
| 30 | 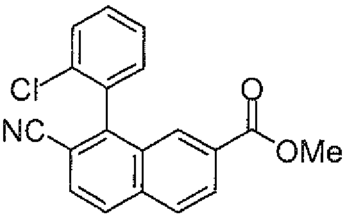 |
| 31 | 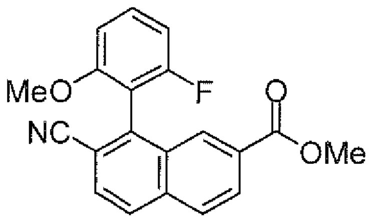 |
| 32 | 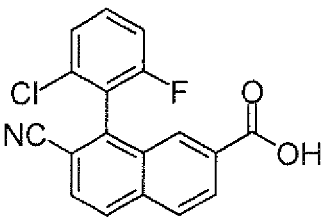 |
| 33 | 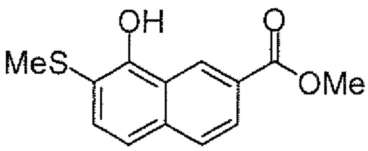 |

| REx | Str |
|---|---|
| 34 | 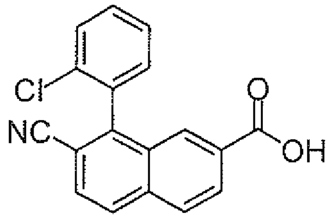 |
| 35 | 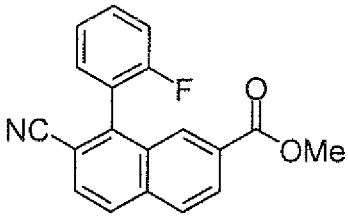 |
| 36 | 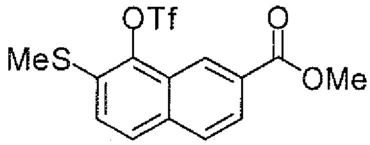 |
| 37 | 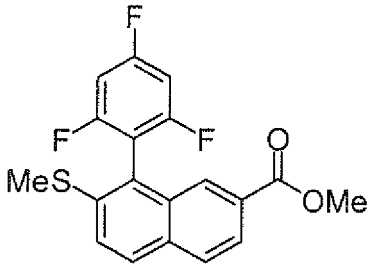 |
| 38 | 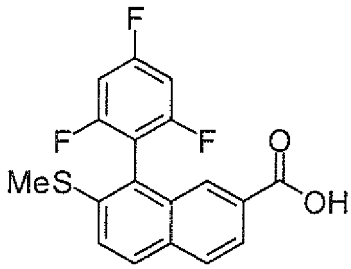 |
| 39 | 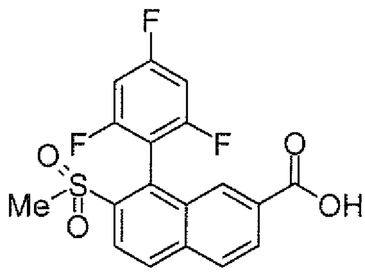 |

[Table 4]

| REx | Str |
|---|---|
| 40 | 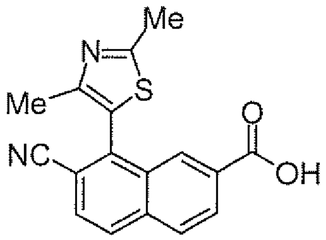 |
| 41 | 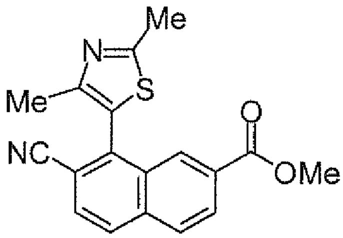 |
| 42 | 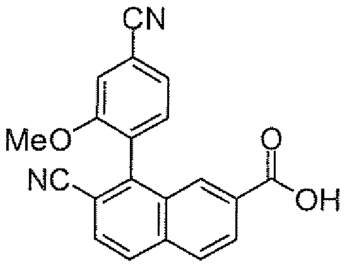 |
| 43 | 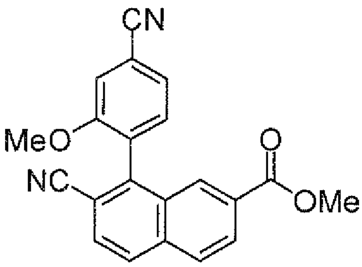 |
| 44 | 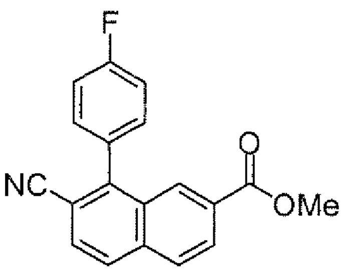 |
| 45 | 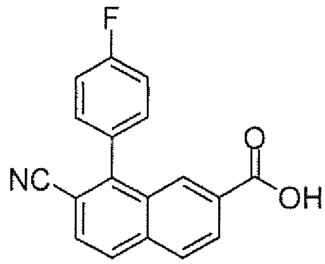 |
| 46 | 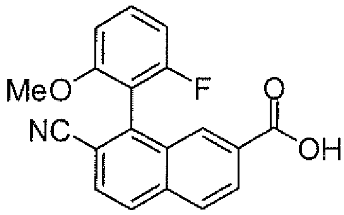 |
| 47 | 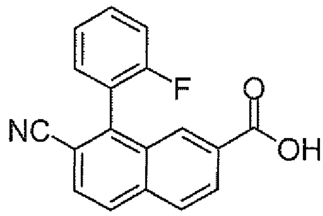 |
| 48 | 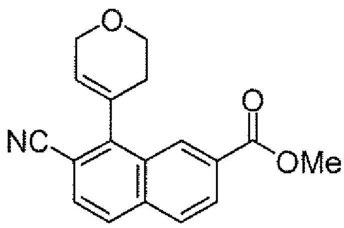 |
| 49 | 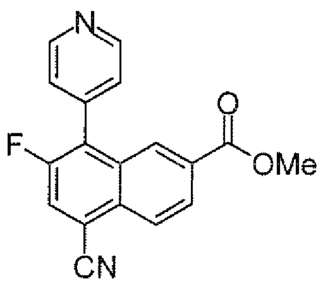 |
| 50 | 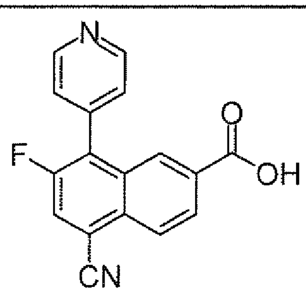 |

[Table 5]

| REx | Str |
|---|---|
| 51 | 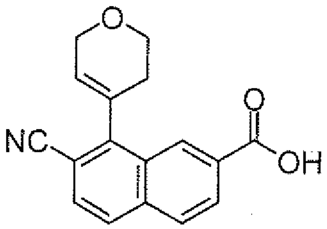 |
| 52 | 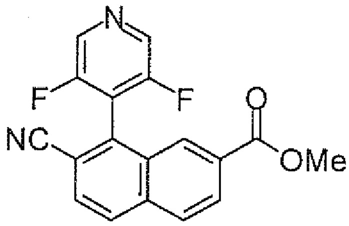 |
| 53 | 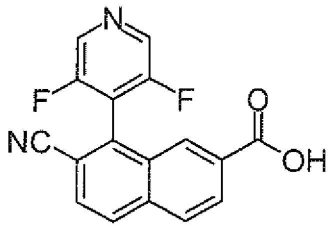 |
| 54 | 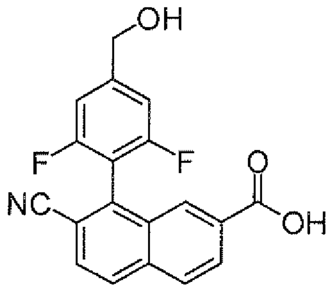 |

| REx | Str |
|---|---|
| 55 | 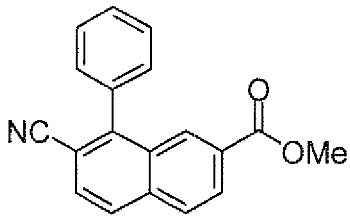 |
| 56 | 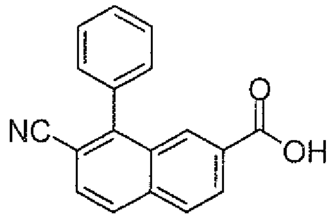 |
| 57 | 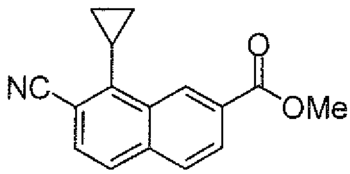 |
| 58 | 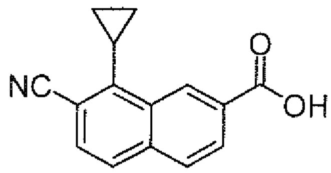 |

[Table 6]

| REx | RSyn | Dat | | REx | RSyn | Dat |
|---|---|---|---|---|---|---|
| 1 | 1 | ESI+: 301, 303 | | 29 | 5 | ESI-: 308 |
| 2 | 2 | ESI+: 221 | | 30 | 13 | ESI+: 322 |
| 3 | 3 | A/E+: 395 | | 31 | 13 | ESI+: 336 |
| 5 | 5 | ESI-: 308 | | 32 | 5 | ESI-: 324 |
| 6 | 6 | ESI-: 297, 299 | | 33 | 1 | ESI+: 249 |
| 7 | 7 | ESI+: 251 | | 34 | 5 | ESI+: 308 |
| 8 | 8 | ESI+: 360 | | 35 | 13 | ESI+: 306 |
| 9 | 9 | ESI+: 228 | | 36 | 8 | FAB+: 381 |
| 10 | 10 | EI: 337 | | 37 | 13 | EI+: 362 |
| 11 | 11 | ESI+: 223 | | 38 | 5 | A/E+: 348 |
| 12 | 12 | EI+: 241 | | 39 | 5 | A/E+: 381 |
| 13 | 13 | EI: 323 | | 40 | 5 | ESI+: 309 |
| 14 | 14 | A/E+: 352 | | 41 | 13 | ESI+: 323 |
| 15 | 1 | A/E+: 283, 285 | | 42 | 5 | ESI-: 327 |
| 16 | 1 | ESI+: 361, 363 | | 44 | 13 | EI: 305 |
| 17 | 2 | ESI-: 279, 281 | | 45 | 5 | ESI-: 290 |
| 18 | 13 | ESI+: 342 | | 46 | 5 | ESI-: 320 |
| 19 | 5 | ESI+: 328 | | 47 | 5 | ESI-: 290 |
| 20 | 13 | ESI+: 323 | | 48 | 13 | EI+: 293 |
| 21 | 5 | ESI+: 309 | | 49 | 13 | ESI+: 306 |
| 22 | 13 | ESI+: 307 | | 50 | 5 | ESI+: 293 |
| 23 | 5 | ESI+: 293 | | 51 | 5 | ESI-: 278 |
| 24 | 9 | ESI-: 244 | | 53 | 5 | ESI-: 309 |
| 25 | 8 | ESI+: 378 | | 54 | 5 | |
| 26 | 13 | ESI+: 360 | | 55 | 13 | A/E-: 287 |
| 27 | 5 | ESI-: 344 | | 56 | 5 | A/E-: 272 |
| 28 | 13 | EI: 323 | | 58 | 5 | ESI+: 238 |

[Table 7]

| REx | RSyn | Dat |
|---|---|---|
| 4 | 4 | NMR-$CDCl_3$: 3.95 (3H, s), 4.85 (2H, s), 7.15 (1H, d, J = 8 Hz), 7.21-7.27 (1H, m), 7.60 (1H, d, J = 12 Hz), 8.10 (1H, d, J = 12 Hz), 8.31 (1H, d, J = 12 Hz), 8.40 (2H, m) |
| 43 | 13 | NMR-$CDCl_3$: 3.74 (3H, s), 3.94 (3H, s), 7.30-7.38 (2H, m), 7.49 (2H, d, J = 8 Hz), 8.36-8.39 (1H, m), 8.07 (1H, d, J = 8 Hz), 8.27-8.29 (2H, m) |
| 52 | 13 | NMR-$CDCl_3$: 3.96 (3H, s), 7.62 (1H, d, J = 8 Hz), 8.13 (1H, d, J = 8 Hz), 8.27-8.28 (1H, m), 8.34-8.37 (1H, m), 8.42-8.44 (1H, m), 8.61 (2H, s) |
| 57 | 13 | NMR-DMSO$d_6$: 0.87-0.93 (2H, m), 1.18-1.25 (2H, m), 2.61-2.66 (1H, m), 3.97 (3H, s), 7.45 (1H, d, J = 7.6 Hz), 8.18-8.28 (3H, m), 9.14 (1H, s) |

[Table 8]

| Ex | Sal | Str |
|---|---|---|
| 1 | HCl | 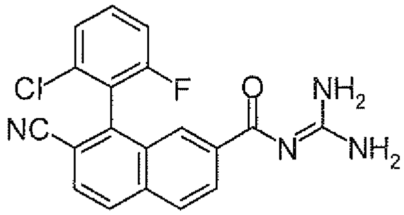 |
| 2 | HCl | 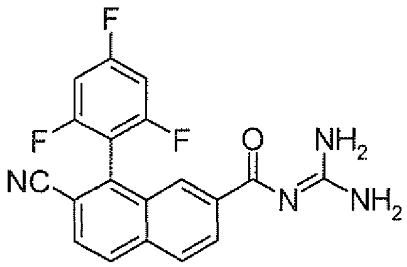 |
| 3 | 2HCl | 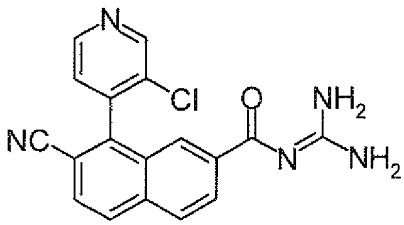 |
| 4 | 2HCl | 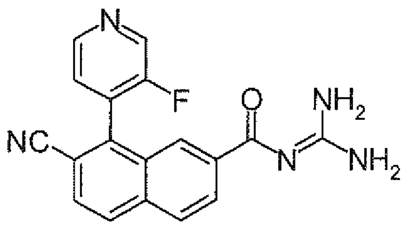 |
| 5 | HCl | 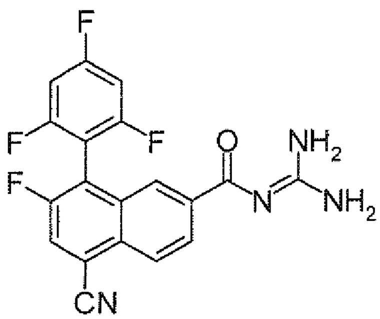 |
| 6 | HCl | 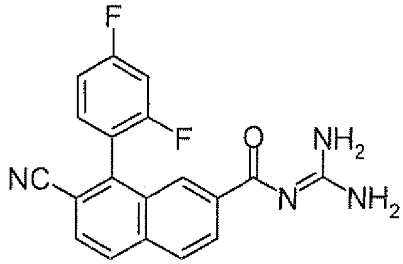 |
| 7 | HCl | 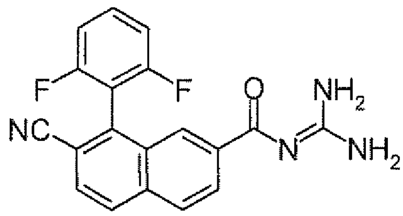 |
| 8 | HCl | 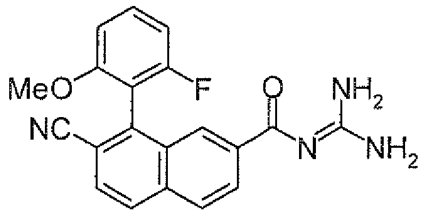 |
| 9 | HCl | 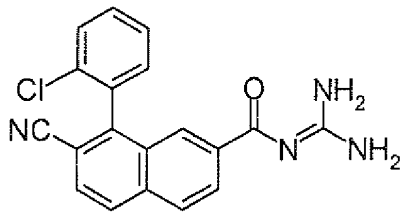 |
| 10 | HCl | 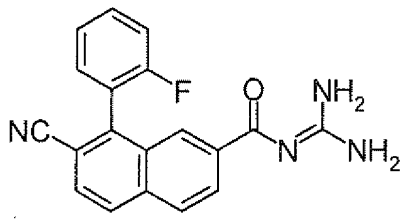 |
| 11 | 2HCl | 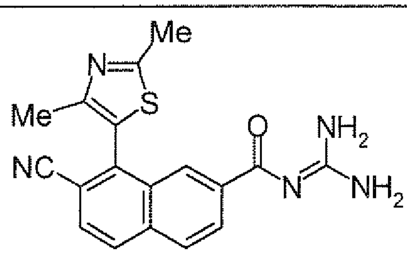 |

[Table 9]

| Ex | Sal | Str |
|---|---|---|
| 12 | HCl | 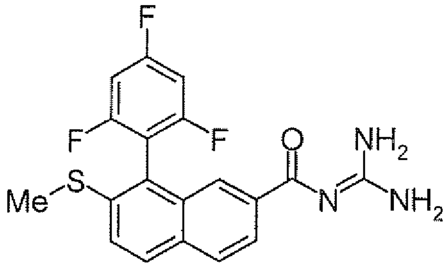 |
| 13 | HCl | 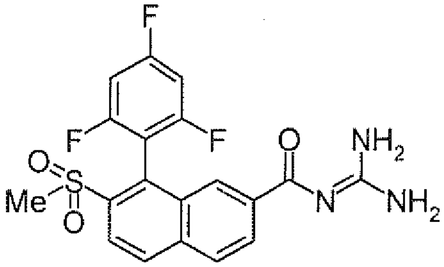 |
| 14 | HCl | 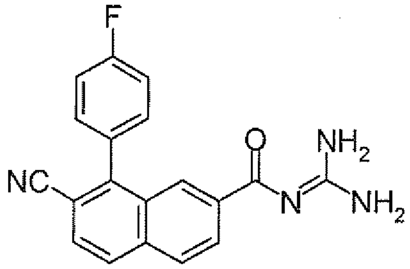 |
| 15 | HCl | 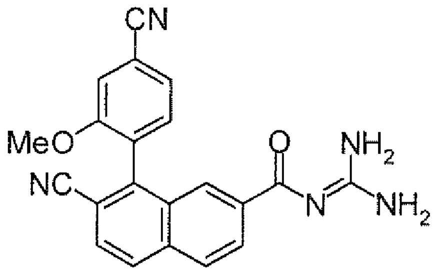 |
| 16 | HCl | 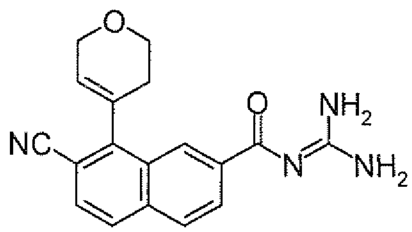 |
| 17 | HCl | 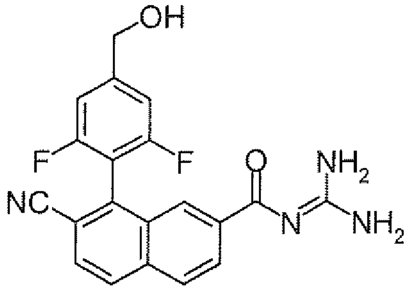 |
| 18 | HCl | 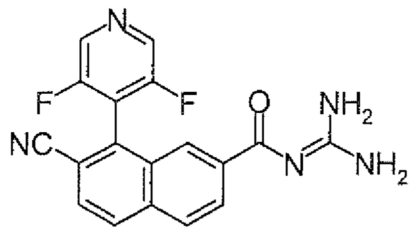 |
| 19 | 2HCl | 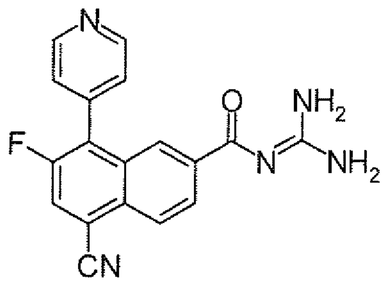 |
| 20 | HCl | 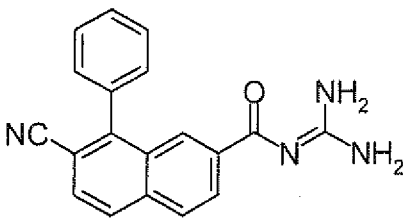 |
| 21 | HCl | 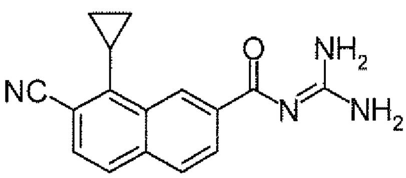 |

[Table 10]

| Ex | Dat |
|---|---|
| 1 | ESI+: 367;<br>NMR-DMSOd$_6$: 7.51 (1H, t, J = 8 Hz), 7.62 (1H, d, J = 8 Hz), 7.66-7.72 (1H, m), 7.86 (1H, d, J = 8 Hz), 8.15 (1H, s), 8.41 (1H, d, J = 8 Hz), 8.49-8.54 (2H, m), 8.59 (4H, brs) |
| 2 | ESI+: 369;<br>NMR-DMSOd$_6$: 7.44 (2H, m), 7.91 (1H, d, J = 7.5 Hz), 8.36-8.73 (8H, m), 12.45 (1H, brs) |
| 3 | ESI+: 350 |
| 4 | ESI+: 334 |
| 5 | ESI+: 387;<br>NMR-DMSOd$_6$: 7.54 (2H, t, J = 8.8 Hz), 8.36-8.80 (8H, m), 12.45 (1H, brs) |
| 6 | ESI+: 351 |
| 7 | ESI+: 351;<br>NMR-DMSOd$_6$: 7.39 (2H, t, J = 8 Hz), 7.70-7.74 (1H, m), 7.91 (1H, d, J = 8 Hz), 8.25 (1H, s), 8.41-8.49 (7H, m), 12.04 (1H, brs) |
| 8 | ESI+: 363 |
| 9 | ESI+: 349, 351 |
| 10 | ESI+: 333 |
| 11 | ESI+: 350 |
| 12 | ESI+: 390 |
| 13 | ESI+: 422 |
| 14 | ESI+: 333 |
| 15 | ESI+: 370 |
| 16 | ESI+: 321 |
| 17 | ESI+: 381 |
| 18 | ESI+: 352;<br>NMR-DMSOd$_6$: 7.98-8.00 (1H, m), 8.41-8.44 (1H, m), 8.47-8.52 (3H, m), 8.58 (2H, brs), 8.75 (2H, brs), 8.78 (2H, s), 12.57 (1H, brs) |
| 19 | ESI+: 334 |
| 20 | ESI+: 315 |
| 21 | ESI+: 279 |

(Test Examples)

Pharmacological activities of compound of the present invention were confirmed by the following tests.

Test Example 1: Acquisition of HEK293 cells for forced expressions of a human 5-HT$_{5A}$ receptor

The ORF (open reading frame; protein coding region) of a human 5-HT$_{5A}$ receptor (Genbank AF498985) was cloned from a human hippocampus cDNA library, and then inserted into a pCR2.1 vector (Invitrogen), and *Escherichia coli* containing the plasmid was cultured in a large amount. Next, the full-length cDNA sequence of the human 5-HT$_{5A}$ receptor was analyzed, and recombined into a pCDNA3.1 vector (Invitrogen) as an expression vector and cultured in a large amount. HEK293 established cells (ATCC) derived from the human fetal kidney were seeded, the expression plasmid (1 μg) obtained above were added thereto with LIPOFECTAMINE 2000 (Invitrogen; 2 μl), the gene was trans-

fected into HEK293 cells, and the expression cells were screened with a drug-resistant marker, Geneticin (G418 sulfate 500 μg/ml; Kanto Chemical Co., Inc.). Thus prepared recombinant cells which expressed the gene were cultured in a medium containing D-MEM (Dulbecco's modified eagle medium, Sigma), 10% FCS (Fetal calf serum: fetal bovine serum), 1% Pc./Sm (Penicillin/Streptomycin, Invitrogen), and 500 μg/ml G418 for 3 days. These experimental operations followed a manual for gene operation experiment and an instruction appended in a reagent, and the like, such as a known method (Sambrook, J. et al, Molecular Cloning-A Laboratory Manual", Cold Spring Harabor laboratory, NY, 1989).

Test Example 2: Test on a human 5-$HT_{5A}$ receptor binding inhibition

(1) Preparation of a membrane from HEK293 cells for forced expressions of a human 5-$HT_{5A}$ receptor

HEK293 cells for forced expressions of a human 5-$HT_{5A}$ receptor were cultured in a F500 plate, and scraped with a scraper. After centrifugation, the precipitate was collected, and an incubation buffer (50 mM Tris (HCl) (pH 7.4), 10 mM $MgSO_4$ and 0.5 mM EDTA (ethylenediamine tetraacetic acid)) was added thereto. After homogenization, it was further centrifuged, and the incubation buffer was added to the precipitate, followed by thoroughly suspending. The operation was repeated, and protein concentration was measured, thereby completing preparation of the membrane.

(2) Test on a human 5-$HT_{5A}$ receptor binding inhibition

A solution of the compound to be tested and 100 μM 5-CT (5-carboxamidetriptamine) in DMSO was added to a 96-well plate at 2 μl/well, suspended in an incubation buffer, and a membrane from HEK293 cells for forced expressions of a human 5-$HT_{5A}$ receptor prepared at 200 μg/ml was added at 100 μl/well. After incubation at room temperature for 15 minutes, a [$^3$H]5-CT solution (2 nM [$^3$H]5-CT, incubation buffer) was added thereto at 100 μl/well.
Separately, 100 μl of the solution was distributed into a liquid scintillation vial, and 2 ml of Aquasol II (registered trademark) was added thereto, followed by stirring. Then, radioactivity was measured by a liquid scintillation counter. It was incubated at 37°C for 60 minutes. The reaction mixture was sucked into 96-well GF/C filter plate that had been pre-treated with 0.2% polyethyleneimine, and washed six times with an ice-cooled, 50 mM Tris (pH 7.5) buffer. The GF/C filter plate was dried.
Microscint TMPS (registered trademark) was added thereto at 40 μl/well. Radioactivity remaining on the GF/C filter plate was measured by a top counter.
The [$^3$H]5-CT binding inhibiting activity by the compound to be tested in each experiment was determined as an $IC_{50}$ value with a radioactivity upon addition of DMSO alone being 0% inhibition, and a radioactivity upon addition of 1 μM 5-CT being 100% inhibition. Separately, Ki values were calculated from the Kd value of the [$^3$H]5-CT determined from Scatchard analysis, by the following equation.

$$Ki = IC_{50}\ (1+\text{Concentraion of ligand added/Kd}\ (4.95\ \text{nM}))$$

As a result, it was demonstrated that compound of formula (I) as an active ingredient of the medicine of the present invention has a potent human 5-$HT_{5A}$ receptor binding inhibiting activity.
For example, the compound of Example 1 gave a Ki value of 4.3nM. Furthermore, the compounds of Examples 2, 5-10, 12, 14, 18 and 20 gave Ki values ranging between 1 nM and 30 nM respectively, and the compounds of Examples 3, 4, 13, 15-17, 19 and 21 gave Ki values ranging between 30 nM and 300 nM respectively.
As described above, it was confirmed that compound of formula (I) has a 5-$HT_{5A}$ receptor affinity.

Test Example 3: Improvement effect on increase in motion induced by methamphetamine or MK-801 in mice.

The improvement effect of compound of formula (I) was evaluated by measuring the quantity of motion by IR irradiation when a compound was administered to a mouse in which hyperactivity was caused by methamphetamine (hereinafter, simply referred to as "MAP") or MK-801, known as an animal model of schizophrenia.

(1) Animal

Species: Male ICR mouse

(2) Operation procedure

An animal was taken out of a breeding cage, orally administered with a compound to be tested, and then placed into a cage for breeding. After 30 minutes, the animal was put into a cage for measurement, and the motion with the compound to be tested alone was measured. Further, after 30 to 90 minutes, the animal was taken out, and intraperitoneally administered with a drug for increasing the motion (MAP; 1 mg/kg or MK-801; 0.3 mg/kg, dissolved in a physiological saline, respectively). Then, the motion for a certain period of time (60 minutes) was measured by using a motion measurement device (CompACTAMS from Muromachi Kikai Co., Ltd.) by means of an infrared sensor.

(3) Analysis

For a normal mouse (a mouse administered with physiological saline) and a mouse administered with a drug for increasing the motion, a Student's T test was performed for evaluation for each interval. For a group administered with the compound to be tested, an assay was performed using a solvent (vehicle) group and a Dunnett's T test. For the evaluation, if there was a significant difference ($P<0.05$), it was considered that there is an effect.
As a result, compound of formula (I) was demonstrated to inhibit the increase in the motion of the mouse induced by the drug. For example, the compound of Example 1 significantly inhibited the hyperactivity caused by MK-801 at a dose of 0.03 mg/kg.
As described above, it was confirmed that compound of formula (I) has an effect of improving schizophrenia.
In addition, the pharmacological effects of compound of formula (I) used as an active ingredient of the medicine of the present invention can be confirmed from the following Test Examples 4 to 6.

Test Example 4: Improvement effect for spontaneous alternation behavior caused by Scoporamine or MK-801 in mice

Effect of compound of formula (I) on improvement on cognitive impairment can be evaluated by using a known performance test method as a model with short-term learning disorder.

(1) Animal

Species: Male ddY mouse

(2) Measurement method

A mouse is placed at the end of one arm of a Y-maze having arms with the same length in three directions, and then explored freely and the number of arm entries is counted for 8 minutes. Furthermore, spontaneous alternation behavior is defined as entries into all three different arms on consecutive occasions. The ratio of the number of this behavior to the total number of entries is calculated as an alternation rate by the following formula:

$$\text{Alternation rate (\%)} = \text{Number of spontaneous alternation behaviors} / (\text{Total number of entries} - 2) \times 100.$$

The compound to be tested is orally administered 50 minutes prior to test, and after 30 minutes, 0.5 mg/kg scopolamine or 0.15 mg/kg MK-801 (in the case of a normal group, physiological saline is administered) is intraperitoneally administered. In addition, a vehicle is orally administered to the normal group (to which physiological saline is administered) and a control group (to which 0.5 mg/kg scopolamine or 0.15 mg/kg MK-801 was administered), when the compound to be tested is administered thereto. Physiological saline is intraperitoneally administered to the normal group, when scopolamine is administered thereto.

(3) Data analysis

If a significant difference between the normal group and the control group (Student's t test) is approved in the alternation rate (%), it is considered to have learning disorder by the administration of Scoporamine or MK-801, By carrying out a Dunnett's test on the group administered with the compound to be tested relative to the control group, the presence or absence of improvement effect of the compound to be tested on learning disorder is evaluated. For each assay, it is considered that there was a significant difference when $p<0.05$.
As a result of the test, it can be confirmed that compound of formula (I) has an effect on cognitive impairment.

Test Example 5: Improvement effect for a disorder of PCP-induced prepulse inhibition (PPI) in rats

When a sound stimulus is given to a human, a startle reaction occurs, but for a normal human, this startle reaction is inhibited when the sound stimulus is preceded by a weak sound stimulus. This inhibiting action is lowered in a patient with schizophrenia. It is known that when a rat is administered with PCP (phencyclidine), a similar symptom to human schizophrenia occurs and is known as a disease model for evaluating information processing disorder as a cognitive impairment of schizophrenia.
Effect of compound of formula (I) on improvement of schizophrenia is evaluated by using this model with prepulse inhibition disorder caused by PCP. Specifically, it follows the method as described in "Neuropsychopharmacology, 1989; 2: 61-66, Mansbach, R.S. and Geyer, M.A. and Brain Research, 1998; 781: 227-235".
As a result of this test, it can be confirmed that compound of formula (I) also has an effect on information processing disorder included in cognitive impairment of schizophrenia.

Test Example 6: Evaluation for water maze learning disorder in old rats

An effect of compound of formula (I) on improvement of dementia is evaluated by using a model with water maze learning disorder known as a model for dementia. Specifically, it follows the method described in J Pharmacol Exp Ther, 1996; 279: 1157-73, Yamazaki M. et al.
As a result of this test, it can be confirmed that compound of formula (I) also has an effect for dementia.
The test results of Test Examples 1 to 6 show that compounds of the present invention are useful for treating or preventing diseases, in which 5-$HT_{5A}$ is concerned, for example treating or preventing dementia, schizophrenia (including symptoms such as positive symptoms, negative symptoms, cognitive impairment and mood disorders), bipolar disorder, attention deficit hyperactivity disorder, psychological disorders (such as panic disorder and obsessive disorder), autism, mood disorders (including anxiety disorder and depression disorder), somnipathy, neurodegenerative diseases and cerebral infarction.
A pharmaceutical preparation containing one or two or more kinds of compound offormula (I) or a salt thereof as an active ingredient can be prepared by using pharmaceutical carriers, excipients, and the like that are each usually used in the art, by a method that is usually used.
Administration may be made in any form for either oral administration by tablets, pills, capsules, granules, powders, and solutions, or parenteral administration by injections for intraarticular injection, intravenous injection, and intramuscular injection, suppositories, ophthalmic solutions, ophthalmic oinments, percutaneous liquids, oinments, percutaneous patches, transmucosal liquids, transmucosal patches, and inhalations.
Regarding the solid composition for oral administration according to the present invention, tablets, powders, granules, or the like are used. In such a solid composition, one, or two or more active ingredients are mixed with at least one inactive excipient such as lactose, mannitol, glucose, hydroxypropyl cellulose, microcrystalline cellulose, starch, polyvinyl pyrrolidone, and/or magnesium meta-silicate alminate. According to a conventional method, the composition may contain inactive additives; for example, a lubricant such as magnesium stearate, a disintegrator such as carboxymethyl starch sodium, a stabilizing agent, and a dissolution promotor. As occasion demands, tablets or pills may be coated with a sugar, or a film of a gastric or enteric materials.
The liquid composition for oral administration includes pharmaceutically acceptable emulsions, solutions, suspensions, syrups, elixirs, and the like, and contains an inert diluent that is commonly used, such as purified water or ethanol. In addition to the inert diluent, this liquid composition may contain an auxiliary agent such as a solubilizing agent, a moistening agent, and a suspending agent, a sweetener, a flavor, an aroma, and an antiseptic.
Injections for parenteral administration include aqueous or non-aqueous sterile solutions, suspensions, and emulsions. Examples of the aqueous solvent include distilled water for injection, and physiological saline. Examples of the non-aqueous solvent include propylene glycol, polyethylene glycol, vegetable oils such as olive oil, alcohols such as ethanol, and Polysorbate 80 (Pharmacopeia). Such a composition may further contain a tonicity agent, an antiseptic, a moistening agent, an emulsifying agent, a dispersing agent, a stabilizing agent, and a dissolution promotor. These are sterilized, for example, by filtration through a bacterium-retaining filter, blending of bactericides, or irradiation. In addition, these can also be used by producing a sterile solid composition, and dissolving or suspending it in sterile water or a sterile solvent for injection prior to its use.
Examples of the drug for external use include ointments, plasters, creams, jellies, cataplasms, sprays, lotions, ophthalmic solutions, and ophthalmic ointments. The drug contains commonly used ointment bases, lotion bases, aqueous or non-aqueous solutions, suspensions, emulsions, and the like. Examples of the ointment bases or lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, and sorbitan sesquioleate.
A transmucosal agent such as an inhalations and a transmucosal agent can be used in a solid, liquid or semi-solid state, and may be produced in accordance with a conventionally known method. For example, a known excipient, and also a

pH adjusting agent, an antiseptic, a surfactant, a lubricant, a stabilizer, a viscosity-increasing agent, and the like may be appropriately added thereto. For their administration, an appropriate device for inhalation or blowing may be used. For example, a compound may be administered alone or as a powder of a formulated mixture, or as a solution or suspension by combining it with a pharmaceutically acceptable carrier, using a conventionally known device or sprayer, such as a measured administration inhalation device. The dry powder inhaler or the like may be for single or multiple administration use, and a dry powder or a powder-containing capsule may be used. Alternatively, this may be in a form such as a high pressure aerosol spray which uses an appropriate propellant, for example, a suitable gas such as chlorofluoroalkane, hydrofluoroalkane, or carbon dioxide.

It is suitable that the daily dose is usually from about 0.0001 to 100 mg/kg per body weight in the case of oral administration, preferably 0.0001 to 10 mg/kg, and even more preferably 0.0001 to 1 mg/kg, and the preparation is administered in one portion or dividing it into 2 to 4 portions. Also, in the case of intravenous administration, the daily dose is administered suitably in a range from about 0.00001 to 1 mg/kg per body weight, and the preparation is administered once a day or two or more times a day. In the case of drugs for external use or transmucosal administration, the drug is administered usually in a range from about 0.0001 to 10 mg/kg per body weight, once a day or two or more times a day. The dose is appropriately decided, depending on individual cases by taking into consideration the symptom, age, sex and the like. The content of the active ingredients in the preparation is from 0.0001 to 50%, and more preferably 0.001 to 50%.

Compound of formula (I) can be used in combination with various agents for preventing or treating diseases in which compound of formula (I) is considered to exhibit pharmaceutical effects, as described above. The combined use may be carried out by simultaneous administration, or separate continuous administration or administration at an interval of a desired period. The simultaneous administration may be carried out by administration in the form of a blend or separate formulations.

Industrial Applicability

Compounds of the present invention have potent 5-$HT_{5A}$ receptor modulating action, and excellent pharmacological action based on the 5-$HT_{5A}$ receptor modulating action. Pharmaceutical compositions of the present invention can be used for prevention or treatment of 5-$HT_{5A}$ receptor-mediated diseases, and in particular, for prevention or of dementia, schizophrenia, bipolar disorder, or attention deficit hyperactivity disorder.

## Claims

1. A compound of formula (I) or a pharmaceutically acceptable salt thereof:

[Chem. 8]

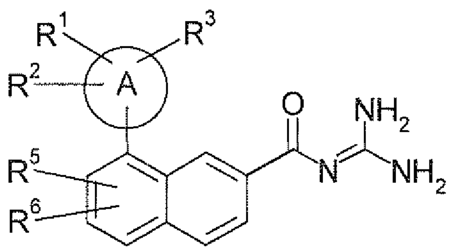

(I)

(wherein symbols have the following meanings:

[Chem. 9]

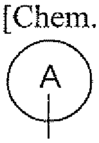

re

presents phenyl, cycloalkyl, monocyclic heteroaryl, or a saturated or partially unsaturated monocyclic oxygen-con-

taining heterocyclic group;
$R^1$, $R^2$ and $R^3$ are the same as or different from each other, and represent H, lower alkyl, halogen, -CN, -$OR^a$, or lower alkylene-$OR^a$;
$R^a$ represents H or lower alkyl;
$R^5$ represents -CN, -SMe, or -$SO_nR^b$;
n represents 1 or 2;
$R^b$ represents lower alkyl; and
$R^6$ represents H or halogen).

**2.** The compound according to claim 1 or a salt thereof, wherein

[Chem. 10]

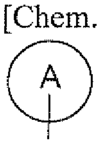

rep

resents phenyl, cyclopropyl, pyridyl, pyrimidinyl, thienyl, thiazolyl, pyrazolyl, oxadiazolyl, tetrahydropyranyl or dihydropyranyl group.

**3.** The compound according to claim 1 or a salt thereof, wherein

[Chem. 11]

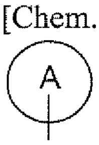

rep

represents phenyl or pyridyl group.

**4.** The compound according to claim 2 or a salt thereof, wherein $R^1$, $R^2$ and $R^3$ are the same as or different from each other, and represent H, methyl, F, Cl, CN, -$OR^a$ or -$CH_2OR^a$.

**5.** The compound according to claim 4 or a salt thereof, wherein the substitution position of $R^5$ is the 5- or 7-position of naphthalene.

**6.** The compound according to claim 5 or a salt thereof, wherein $R^6$ represents H, F or Cl.

**7.** The compound according to claim 1 or a salt thereof, which is selected from the group consisting of
7-cyano-N-(diaminomethylene)-8-(2,4,6-trifluorophenyl)-2-naphtliamide,
7-cyano-N-(diaminomethylene)-8-(2,6-difluorophenyl)-2-naphthamide,
5-cyano-N-(diaminomethylene)-7-fluoro-8-(2,4,6-trifluorophenyl)-2-naphthamide,
8-(2-chloro-6-fluorophenyl)-7-cyano-N-(diaminomethylene)-2-naphthamide, and
7-cyano-N-(diaminomethylene)-8-(3,5-difuoropyridin-4-yl)-2-naphthamide.

**8.** A pharmaceutical composition comprising the compound according to claim 1 or a salt thereof and a pharmaceutically acceptable carrier.

**9.** The pharmaceutical composition according to claim 8, which is a 5-$HT_{5A}$ receptor modulator.

**10.** The pharmaceutical composition according to claim 9, which is an agent for preventing or treating dementia, schizophrenia, bipolar disorder or attention deficit hyperactivity disorder.

**11.** Use of the derivative according to claim 1 or a salt thereof for the manufacture of an agent for preventing or treating

dementia, schizophrenia, bipolar disorder or attention deficit hyperactivity disorder.

**12.** A method for preventing or treating dementia, schizophrenia, bipolar disorder or attention deficit hyperactivity disorder, comprising administering a therapeutically effective amount of the derivative according to claim 1 or a salt thereof to a patient.

# INTERNATIONAL SEARCH REPORT

International application No.
PCT/JP2010/051757

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07C279/22

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| P,A | WO 2009/022633 A1 (Astellas Pharma Inc.), 19 February 2009 (19.02.2009), the entire description (Family: none) | 1-11 |
| A | JP 2008-543886 A (Biotron Ltd.), 04 December 2008 (04.12.2008), the entire specification & US 2009/0099239 A1 & EP 1902017 A1 & WO 2006/135978 A1 & CA 2612403 A & KR 10-2008-0021810 A & CN 101208297 A & ZA 200800256 A | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 March, 2010 (02.03.10) | 16 March, 2010 (16.03.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/051757

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-81664 A (Hoechst AG.),<br>31 March 1998 (31.03.1998),<br>the entire specification<br>& US 6087304 A & EP 810206 A1<br>& DE 19621483 A & DE 59702802 D<br>& NO 972433 A & NZ 314915 A<br>& BR 9703338 A & HU 9700955 A<br>& AU 710065 B & AU 2364597 A<br>& CA 2206366 A & CZ 9701630 A<br>& HR 970292 A & IL 120924 A<br>& PL 318723 A & SK 67097 A<br>& AT 198320 T & TR 9700428 A<br>& ES 2154002 T & DK 810206 T<br>& SI 810206 T & TW 416944 B<br>& PT 810206 E & ZA 9704665 A<br>& RU 2190600 C & ID 16989 A<br>& GR 3035126 T & CN 1167759 A<br>& MX 9703922 A & NO 972433 A0 | 1-11 |

Form PCT/ISA/210 (continuation of second sheet) (July 2009)

**INTERNATIONAL SEARCH REPORT**

International application No.
PCT/JP2010/051757

Continuation of A. CLASSIFICATION OF SUBJECT MATTER
(International Patent Classification (IPC))

*C07C279/22*(2006.01)i, *A61K31/166*(2006.01)i, *A61K31/192*(2006.01)i, *A61K31/216*(2006.01)i, *A61K31/277*(2006.01)i, *A61K31/351*(2006.01)i, *A61K31/426*(2006.01)i, *A61K31/44*(2006.01)i, *A61P25/18*(2006.01)i, *A61P25/28*(2006.01)i, *A61P43/00*(2006.01)i, *C07C317/44*(2006.01)i, *C07C323/62*(2006.01)i, *C07D213/61*(2006.01)i, *C07D277/20*(2006.01)i, *C07D277/56*(2006.01)i, *C07D309/22*(2006.01)i

(According to International Patent Classification (IPC) or to both national classification and IPC)

Form PCT/ISA/210 (extra sheet) (July 2009)

**INTERNATIONAL SEARCH REPORT**

International application No.
PCT/JP2010/051757

**Box No. II Observations where certain claims were found unsearchable (Continuation of item 2 of first sheet)**

This international search report has not been established in respect of certain claims under Article 17(2)(a) for the following reasons:

1. [x] Claims Nos.: 12
because they relate to subject matter not required to be searched by this Authority, namely:
Claim 12 pertains to method for treatment of the human body by therapy and thus relates to a subject matter on which the International Searching Authority is not required to carry out a search under the provisions of PCT Rule 39.1(iv).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 05082871 A
- WO 06135978 A
- WO 04112687 A
- US 6087304 A
- US 6093729 A
- JP 8225513 A
- US 5824691 A

### Non-patent literature cited in the description


- *Neuron,* 1999, vol. 22, 581-591
- *Molecular Brain Research,* 1998, vol. 56, 1-8
- *Neuroreport,* 2000, vol. 11, 2017-2020
- *Mol. Psychiatr.,* 2001, vol. 6, 217-219
- *J. Psychiatr. Res.,* 2004, vol. 38, 371-376
- **Takeshi Yamamoto et al.** Chemical and Pharmaceutical Bulletin. 1997, vol. 45, 1282-1286
- *Prog. Med.,* 1985, vol. 5, 2157-2161
- Iyakuhin no Kaihatsu. Bunshi Sekkei. Hirokawa Publishing Company, 1990, vol. 7, 163-198
- Green's Protective Groups in Organic Synthesis. 2006
- **A. d. Meijere ; F. Diederich et al.** Metal-Catalyzed Cross-Coupling Reactions. VCH Publishers Inc, 1997
- Jikken Kagaku Koza. The Chemical Society of Japan. Maruzen, 2005, vol. 13
- **P. G M. Wuts ; T. W. Greene.** Green's Protective Groups in Organic Synthesis. 2006
- **Sambrook, J. et al.** Molecular Cloning-A Laboratory Manual. Cold Spring Harabor laboratory, 1989
- *Neuropsychopharmacology,* 1989, vol. 2, 61-66
- **Mansbach, R.S. ; Geyer, M.A.** *Brain Research,* 1998, vol. 781, 227-235
- **Yamazaki M.** *J Pharmacol Exp Ther,* 1996, vol. 279, 1157-73